# EUROPEAN PATENT APPLICATION

(11) **EP 4 491 636 A1**
(43) Date of publication of application: **15.01.2025**
(21) Application number: 23767060.9
(22) Date of filing: 24.02.2023
(51) Int. Cl.: C07K 16/44, C07K 14/725, C12N 5/0783, A61K 39/00, A61K 35/17, A61P 37/00, C07K 14/705

(54) **ANTIBODY OR ANTIGEN-BINDING FRAGMENT THEREOF THAT TARGETS COTININE, CHIMERIC ANTIGEN RECEPTOR COMPRISING SAME, AND USES THEREOF**

(30) Priority: 11.03.2022 KR 20220031019; 06.09.2022 KR 20220113082
(71) Applicant: AbClon Inc., Seoul 08381 (KR)
(72) Inventor: LEE, Jong-Seo, Seongnam-Si Gyeonggi-do 13473 (KR); LEE, Young Ha, Seoul 04753 (KR); LEE, Jong-Ho, Anyang-si Gyeonggi-do 13903 (KR); KIM, Ki Hyun, Seoul 06247 (KR)
(74) Representative: J A Kemp LLP
(86) International application number: PCT/KR2023/002676
(87) International publication number: WO 2023/171958

(57) **Abstract**

The present invention relates to an antibody or antigen-binding fragment thereof that targets cotinine, a chimeric antigen receptor comprising same, and uses thereof. The antibody of the present invention is an antibody that specifically binds to cotinine, and in particular, an antibody that binds more specifically to the S-isomer of cotinine than to the R-isomer thereof. In addition, the antibody has very low homology and a unique sequence, compared to the CDR sequences of conventional cotinine target antibodies. Cells expressing a chimeric antigen receptor comprising the anti-cotinine antibody or antigen-binding fragment of the present invention bind to a cotinine-conjugated switch and respond to a target cell line, thereby inducing immune cell activity. Therefore, the antibody or antigen-binding fragment thereof of the present invention can be used to suppress immune side effects due to overactivation of T cells through cotinine-mediated activation regulation of chimeric antigen receptor effector cells, and can be effectively used as a CAR-immune cell therapeutic agent.

## Description

### Technical Field

The present disclosure was made with the support of the Ministry of Trade, Industry, and Energy of the Republic of Korea, under Project Identification Number 1415175509, and Sub-project Number 20002893. The specialized agency for managing the research of the aforementioned project is the Korea Evaluation Institute of Industrial Technology. The name of the research project is "Bio-Industry Core Technology Development Project," and the research task is "Development of HER2 Targeted Ovarian Cancer Therapeutics Using Innovative Switchable CART Technology." The principal organization is AbClon Inc., and the research period is from October 1, 2018, to June 30, 2022.

This patent application claims the benefit of and priority to Korean Patent Application No. 10-2022-0031019, filed on March 11, 2022, and Korean Patent Application No. 10-2022-0113082, filed on September 6, 2022, with the Korean Intellectual Property Office, the disclosures of which are incorporated herein by reference.

The present disclosure relates to an antibody targeting cotinine or an antigen-binding fragment thereof, a chimeric antigen receptor comprising same, and a use thereof.

### Background Art

Throughout this specification, numerous papers and patent documents are referenced and citations are indicated. The disclosures of the cited papers and patent documents are incorporated by reference in their entirety into this specification to more clearly describe the level of skill in the art and the details of the present disclosure.

T-cells, one of the immune cells, are well known to play an important role in cell-mediated immunity in humans and animals. CAR-T therapeutics, which are genetically recombined T-cells, induce cell death of target cells (such as cancer cells) by introducing receptor genes that specifically recognize target molecules expressed in the target cells, thereby activating the cytotoxicity of T-cells. Specifically, CAR-T therapeutics have been recognized as a dream cancer treatment, showing about 80% treatment rate in early clinical trials for leukemia patients who were unresponsive to all existing treatments by reactivating the weakened immune system. In August 2017, Novartis' Kymriah and in October 2017, Kite Pharma, a subsidiary of Gilead, received FDA approval for Yescarta, leading to commercialization.

However, despite such early-stage successful clinical efficacy, conventional CAR-T cells face persistent safety concerns due to the inability to control their activation and expansion in vivo. For example, CAR-T cells can persist in the body for a long time, raising potential risks, and can cause severe symptoms due to cytokine release syndrome (CRS). To overcome the issues of existing CAR-T therapeutics, switchable CAR-T, which is a next-generation CAR-T platform, has been newly developed by incorporating a switch function to control the activity of CAR-T cells.

In the switchable CAR-T system, a substance which can be recognized by the chimeric antigen receptor of CAR-T and can also recognize target molecules is used as a mediator that functions as a switch. For example, recent studies have constructed a switchable CAR-T system by combining cotinine as an adapter with antibodies used in existing cancer immunotherapy and utilizing same as a switch molecule, whereby advantage can be taken of conventional antibodies that specifically bind to cotinine. Through the ON/OFF mechanism of the switch molecule, the system has enabled the control of CAR-T cell activation that has faced a challenge. Furthermore, combining cotinine with antibodies used in existing cancer immunotherapy has made it possible to create a multi-targeting CAR-T system using various switch molecules in the form of cotinine-bound molecules.

Cotinine, a metabolite of nicotine, is highly useful as a component of switch molecules because it is not biosynthesized in the body and does not induce physiological activity. However, to improve the switchable CAR system using cotinine as an adapter for switch molecules, it is necessary to develop improved cotinine-specific antibodies that can induce superior cytotoxic activity.

### Disclosure of Invention

### Technical Problem

Leading to the present disclosure, intensive and thorough research conducted by the present inventors into the development of a novel antibody capable of inducing superior cytotoxic activity compared to existing cotinine-targeting antibodies, and a chimeric antigen receptor utilizing the novel antibody resulted in successfully developing a novel antibody or its antigen-binding fragment with very low homology compared to the CDR sequences of conventional cotinine-targeting antibodies. Furthermore, chimeric antigen receptors using humanized antibodies of the antibody and effector cells expressing the chimeric antigen receptors were created and found to effectively induce immune cell activation at high concentrations compared to conventional cotinine-targeting antibodies.

Therefore, the present disclosure aims to provide an antibody or antigen-binding fragment thereof that each bind more specifically to the S-isomer of cotinine.

Also, the present disclosure is to provide a chimeric antigen receptor comprising the antibody or antigen-binding fragment thereof, and an effector cell of the receptor.

Furthermore, the present disclosure is to provide an immunotherapeutic pharmaceutical composition comprising the effector cell and a switch molecule for controlling the activation of the effector cell, and a pharmaceutical kit thereof.

### Solution to Problem

The present disclosure provides the invention as set forth in the following 1 to 27:
1. An antibody or an antigen-binding fragment thereof that binds more specifically to the S-isomer of cotinine than to the R-isomer of cotinine.
2. The antibody or antigen-binding fragment according to 1, wherein the antibody or antigen-binding fragment comprises: a heavy chain variable region comprising HCDR1 with the amino acid sequence of SEQ ID NO: 1, HCDR2 with the amino acid sequence of SEQ ID NO: 2, and HCDR3 with the amino acid sequence of SEQ ID NO: 3; and
   a light chain variable region comprising LCDR1 with the amino acid sequence of SEQ ID NO: 4, LCDR2 with the amino acid sequence of SEQ ID NO: 5, and LCDR3 with the amino acid sequence of SEQ ID NO: 6.
3. The antibody or antigen-binding fragment according to 1, wherein the antibody or antigen-binding fragment comprises:
   (i) a heavy chain variable region comprising the amino acid sequence of SEQ ID NO: 7 and a light chain variable region comprising the amino acid sequence of SEQ ID NO: 8; or
   (ii) a heavy chain variable region comprising the amino acid sequence of SEQ ID NO: 9 and a light chain variable region comprising the amino acid sequence of SEQ ID NO: 10.
4. The antibody or antigen-binding fragment according to 1, wherein the antibody or antigen-binding fragment is selected from the group consisting of monoclonal antibody, polyclonal antibody, human antibody, humanized antibody, chimeric antibody, scFv, Fab, F(ab), and F(ab')₂, each comprising a heavy chain variable region and a light chain variable region.
5. A nucleic acid molecule comprising a nucleotide sequence encoding the antibody or antigen-binding fragment according to any one of 1 to 4.
6. The nucleic acid molecule according to 5, wherein the nucleic acid molecule comprises:
   (i) a nucleotide sequence of SEQ ID NO: 12 encoding a heavy chain variable region and a nucleotide sequence of SEQ ID NO: 13 encoding a light chain variable region; or
   (ii) a nucleotide sequence of SEQ ID NO: 14 encoding a heavy chain variable region and a nucleotide sequence of SEQ ID NO: 15 encoding a light chain variable region.
7. A recombinant vector comprising the nucleic acid molecule of 5.
8. A host cell comprising the recombinant vector of 7.
9. A chimeric antigen receptor comprising the antibody or antigen-binding fragment that binds more specifically to the S-isomer of cotinine according to any one of 1 to 4.
10. A chimeric antigen receptor effector cell expressing a chimeric antigen receptor comprising the antibody or antigen binding fragment thereof that binds more specifically to the S-isomer of cotinine according to any one of 1 to 4.
11. The chimeric antigen receptor effector cell according to 10, wherein the activity of the chimeric antigen receptor effector cell is regulated by a switch molecule comprising:
   (a) the S-isomer of cotinine; and
   (b) a targeting moiety that binds to a cell surface molecule on a target cell.
12. The effector cell according to 11, wherein the cell surface molecule on the target cell is selected from the group consisting of HER2, CD19, EGFR, VEGFR2, CD80, CD86, mesothelin, PSMA (prostate specific membrane antigen), CD20, MUC1, and combinations thereof.
13. The chimeric antigen receptor effector cell according to 11, wherein the targeting moiety comprising the switch molecule for regulating activation is an antibody, an antigen-binding fragment of an antibody, or a target-binding polypeptide.
14. The chimeric antigen receptor effector cell according to 13, wherein the switch molecule for regulating activation is a cotinine-conjugated affibody comprising the S-isomer of cotinine.
15. An immunotherapeutic pharmaceutical composition comprising the chimeric antigen receptor effector cell according to 10 as an active ingredient.
16. An immunotherapeutic pharmaceutical kit comprising the chimeric antigen receptor effector cell according to any one of items 10 to 14 as an active ingredient.
17. A method comprising administering the chimeric antigen receptor effector cell according to any one of 10 to 14 and a switch molecule binding to the chimeric antigen receptor to a subject in need of treatment.
18. The method according to 17, wherein the method is for treating a disease or condition related to a tumor or cancer.
19. The method according to 17, wherein the method is for treating a disease or condition related to autoimmunity.
20. A method comprising the steps of: administering the chimeric antigen receptor effector cell according to any one of 10 to 14 and a switch molecule binding to the chimeric antigen receptor to a subject in need of treatment; and
   inhibiting the activity of the chimeric antigen receptor effector cell.
21. The method according to 20, wherein the method inhibits side effects due to the administration of the chimeric antigen receptor effector cell.
22. The method according to 20, wherein the step of inhibiting the activity of the chimeric antigen receptor effector cell comprises adding a substance that binds to the chimeric antigen receptor.
23. The method according to 22, wherein the substance binding to the chimeric antigen receptor is cotinine, preferably S-cotinine.
24. The method according to 20, wherein the step of inhibiting the activity of the chimeric antigen receptor effector cell comprises adding a substance that binds to the switch molecule.
25. The method according to 24, wherein the substance binding to the switch molecule is an isolated antibody or antigen-binding fragment that binds to cotinine.
26. The method according to 24, wherein the substance binding to the switch molecule is an isolated cell surface molecule of the target cell that binds to the targeting moiety of the switch molecule.
27. A chimeric antigen receptor-effector cell therapeutic system comprising the chimeric antigen receptor effector cell according to any one of 10 to 14 and a switch molecule binding to the chimeric antigen receptor.

The present inventors have developed an antibody or an antigen-binding fragment thereof that can each induce superior cytotoxic activity compared to conventional cotinine-targeting antibodies.

In particular, a chimeric antigen receptor effector cell comprising the novel antibody or antigen-binding fragment thereof targeting cotinine of the present disclosure have been confirmed to induce effective immune cell activation at high concentrations, depending on the isomer type of cotinine, compared to conventional cotinine-targeting antibodies.

According to an aspect thereof, the present disclosure provides an antibody or an antigen-binding fragment thereof that each bind more specifically to the S-isomer of cotinine than to the R-isomer of cotinine.

As used herein, the term "cotinine" refers to the main metabolite of nicotine, which has the structure of Chemical Formula 1:

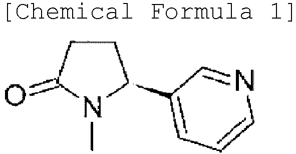

Being neither biosynthesized de novo nor inducing physiological activity, cotinine does not alter the properties of the conjugated substance. Also, cotinine can be bound by an antibody that has specific affinity therefor. In addition, cotinine is easy to prepare into a carboxyl group-linkeded form which allows for conjugation with a substance.

Cotinine has stereoisomers classified as the S-isomer and the R-isomer.

The term "isomer", as used herein, refers to molecules that have the same molecular formula but differ in the arrangement of constituent atoms or their spatial configuration. Isomers can be broadly categorized into constitutional isomers (also known as structural isomers) and stereoisomers. Constitutional isomers have the same molecular formula but differ in the bonding of atoms within the molecule, such as the type, number, and position of bonds or functional groups. Stereoisomers have the same bonding of atoms but differ in the spatial arrangement of atoms, resulting in molecules that are not superimposable. The stereoisomers can be further divided into enantiomers or optical isomers, and diastereomers. Enantiomers refer to stereoisomers that are non-superimposable mirror images of each other. Diastereomers refer to stereoisomers that are not related as mirror images but are still not superimposable. This category includes geometric isomers and conformational isomers.

Therefore, isomers can be distinguished as different compounds with different physical/chemical properties, despite having the same molecular formula. For example, in the body, even with the same physical properties, only one optical isomer may have physiological activity due to specific optical activity-related environments for optical isomers. Thus, the importance of each isomer with different physical/chemical properties is emphasized in terms of their biological or pharmaceutical roles.

Cotinine has stereoisomers, and its isomers are represented by the structures of Chemical Formula **2.** Except for being distinguished by the prefixes "cis-trans" or **"R-S,"** the isomers have the same name and are referred to hereafter as the S-isomer and R-isomer of cotinine in the description.

### [Chemical Formula 2]

| | |
|---|---|
| R-isomer | S-isomer |
| 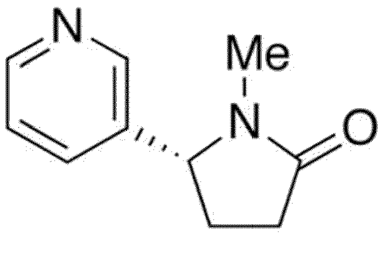 | 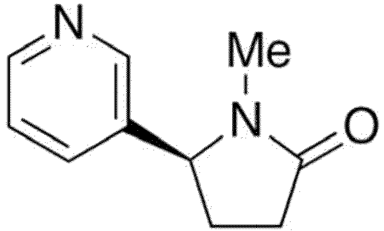 |

| | |
|---|---|
| -Me : -CH₃ | |

In an embodiment of the present disclosure, a cotinine racemate containing a mixture of two isomers was separated into two single isomers using a chiral column, and it was confirmed that the antibody or antigen-binding fragment thereof according to the present disclosure binds more specifically to the S-isomer of cotinine than to the R-isomer.

In an embodiment of the present disclosure, the antibody or antigen-binding fragment thereof that binds more specifically to the S-isomer of cotinine comprises a heavy chain variable region comprising HCDR1 with the amino acid sequence of SEQ ID NO: 1, HCDR2 with the amino acid sequence of SEQ ID NO: 2, and HCDR3 with the amino acid sequence of SEQ ID NO: 3; and a light chain variable region comprisingLCDR1 with the amino acid sequence of SEQ ID NO: 4, LCDR2 with the amino acid sequence of SEQ ID NO: 5, and LCDR3 with the amino acid sequence of SEQ ID NO: 6.

In a specific embodiment of the present disclosure, the antibody or antigen-binding fragment thereof according to the present disclosure, which binds more specifically to the S-isomer of cotinine, comprises (i) a heavy chain variable region with the amino acid sequence of SEQ ID NO: 7 and a light chain variable region with the amino acid sequence of SEQ ID NO: 8; or (ii) a heavy chain variable region with the amino acid sequence of SEQ ID NO: 9 and a light chain variable region with the amino acid sequence of SEQ ID NO: 10.

As used herein, the term "antibody" is intended to encompass not only an intact antibody, but also antigen-binding fragments thereof, which all specifically bind to a particular antigen. An intact antibody has a structure comprising two full-length light chains and two full-length heavy chains, each light chain being linked to the corresponding heavy chain via a disulfide bond. The constant region of an antibody is divided into a heavy chain constant region and a light chain constant region. The heavy chain constant region comprises gamma (γ), mu (µ), alpha (α), delta (δ), and epsilon (ε) types, and subclasses such as gamma1 (γ1), gamma2 (γ2), gamma3 (γ3), gamma4 (γ4), alpha1 (α1), and alpha2 (α2). The light chain constant region comprises kappa (κ) and lambda (λ) types (Cellular and Molecular Immunology, Wonsiewicz, M. J., Ed., Chapter 45, pp. 41-50, W. B. Saunders Co. Philadelphia, PA (1991); Nisonoff, A., Introduction to Molecular Immunology, 2nd Ed., Chapter 4, pp. 45-65, Sinauer Associates, Inc., Sunderland, MA (1984)).

As used herein, the term "antibody" means an antibody that specifically binds to cotinine and comprises not only an intact antibody but also antigen-binding fragments of the antibody molecule. Specifically, as used herein, the term "antibody" refers to an antibody that binds more specifically to the S-isomer of cotinine compared to the R-isomer, including not only an intact antibody but also antigen-binding fragments of the antibody molecule.

As used herein, the term "antigen-binding fragment" means a fragment retaining the function of binding to an antigen and is intended to encompass Fab, F(ab'), F(ab')₂, chemically linked F(ab')₂, Fv, and so on. Of the antibody fragments, Fab has one antigen-binding site which takes on the structure composed of light chain and heavy chain variable domains, a light chain constant domain, and the first constant domain (CH1 domain) of the heavy chain. Fab' is different from Fab in that the former has a hinge region comprising at least one cysteine residue at the C terminus of the heavy chain CH1 domain. An F(ab')₂ antibody is produced in such a way that a cysteine residue of the hinge region of Fab' forms disulfide bonding. Fv is a minimum antibody fragment having only a heavy chain variable domain and a light chain variable domain. A recombination technology for producing an Fv fragment is disclosed in the International Patent Publication filed under the patent cooperation treaty (PCT) WO 88/10649, WO 88/106630, WO 88/07085, WO 88/07086, and WO 88/09344. In case of two-chain Fv, a heavy chain variable domain and a light chain variable domain are linked to each other by means of noncovalent bonding while single-chain Fv consists of a heavy chain variable domain and a single chain variable domain which are linked to each other by means of covalent bonding generally via a peptide linker, or directly linked to each other at C-terminus, and thus may form a structure like a dimer, as shown in the two-chain Fv. Such antibody fragments may be obtained by using protease (for example, Fab may be obtained by performing restriction digestion of a whole antibody with papain, while F(ab')₂ fragment may be obtained by doing so with pepsin), and may be prepared by means of a gene recombination technology.

The antibody or antigen binding fragment thereof in the present disclosure is preferably in the form of scFv or in an intact form. In addition, the heavy chain constant domain may be any one isotype selected from gamma (γ), mu (µ), alpha (α), delta (δ), and epsilon (ε). Preferably, the constant domain may be a gamma 1 (IgG1), gamma 2 (IgG2), gamma 3 (IgG3), or gamma 4 (IgG4) isotype, with most preference for gamma 4 (IgG4) isotype. The light chain constant domain may be a kappa or lambda isotype, with preference for kappa isotype. Therefore, a specific antibody of the present disclosure is in the form of scFv or IgG1 comprising a kappa (κ) light chain and a gamma 4 (IgG4) heavy chain, but with no limitations thereto.

The term "heavy chain", as used herein, refers to a full-length heavy chain comprising: a variable domain V_{H}, which comprises amino acid sequences having enough variable domain sequences to allow the specificity to an antigen; and the three constant domains, CH1, CH2, CH3, and hinge, and to any fragment thereof. As used herein, the term "light chain" refers to a full-length light chain comprising: a variable domain V_{L}, which comprises amino acid sequences having enough variable domain sequences to allow the specificity to an antigen; and a constant domain, C_{L} and to any fragment thereof.

The term "CDR" (complementarity determining region), as used herein, refers to an amino acid sequence in a hypervariable region of an immunoglobulin heavy chain or light chain (Kabat et al., Sequences of Proteins of Immunological Interest, 4th Ed., U.S. Department of Health and Human Services, National Institutes of Health (1987)). Three CDRs exist in each of the heavy chain (HCDR1, HCDR2, and HCDR3) and the light chain (LCDR1, LCDR2, and LCDR3), with framework regions (FR) present between these CDRs to support the CDR loops. CDRs, which are loop-like regions involved in antigen recognition, provide contact residues which play an important role in binding the antibody to an antigen or an epitope, thereby determining the antibody's specificity.

The term "framework" or "FR", as used herein, refers to variable domain residues other than hypervariable region (HVR) residues. The FR of a variable domain generally consists of four FR domains: FR1, FR2, FR3, and FR4.

The term "variable region" or "variable domain" refers to the domain of an antibody heavy or light chain responsible for binding the antibody to antigen. The variable domains of the heavy chain and light chain (VH and VL, respectively) of a native antibody generally have similar structures, with each domain comprising four conserved framework regions (FRs) and three hypervariable regions (HVRs). (Kindt et al. Kuby Immunology, 6th ed., W.H. Freeman and Co., page 91 (2007)). A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively.

The term "specifically binds" or similar expressions mean that an antibody or an antigen-binding fragment thereof, or another construct such as an scFv, interacts with an antigen or a corresponding substance thereto, exhibiting an immunological response. Specific binding can be characterized by an equilibrium dissociation constant of at least about 1×10⁻⁶ M or less (e.g., 9×10⁻⁷ M, 8×10⁻⁷ M, 7×10⁻⁷ M, 6×10⁻⁷ M, 5×10⁻⁷ M, 4×10⁻⁷ M, 3×10⁻⁷ M, 2×10⁻⁷ M, or 1×10⁻⁷ M), preferably 1×10⁻⁷ M or less (e.g., 9×10⁻⁸ M, 8×10⁻⁸ M, 7×10⁻⁸ M, 6×10⁻⁸ M, 5×10⁻⁸ M, 4×10⁻⁸ M, 3×10⁻⁸ M, 2×10⁻⁸ M, or 1×10⁻⁸ M), and more preferably 1×10⁻⁸ M or less (e.g., 9×10⁻⁹ M, 8×10⁻⁹ M, 7×10⁻⁹ M, 6×10⁻⁹ M, 5×10⁻⁹ M, 4×10⁻⁹ M, 3×10⁻⁹ M, 2×10⁻⁹ M, or 1×10⁻⁹ M) (e.g., a smaller K_{D} denotes a tighter binding). Methods for determining whether two molecules specifically bind are well known in the art and include, for example, equilibrium dialysis, surface plasmon resonance, and the like.

The antibody or antigen-binding fragment thereof according to the present disclosure includes not only the full-length or intact polyclonal or monoclonal antibody but also antigen-binding fragments thereof (e.g., Fab, Fab', F(ab')₂, Fab₃, Fv, and variants thereof), fusion proteins comprising one or more antibody portions, human antibodies, humanized antibodies, chimeric antibodies, minibodies, diabodies, triabodies, tetrabodies, linear antibodies, single-chain antibodies (scFv), scFv-Fc, bispecific antibodies, multispecific antibodies, glycosylation variants of antibodies as other modified configurations of immunoglobulin molecules possessing antigen recognition sites of required specificity, amino acid sequence variants of antibodies, and covalently modified antibodies. Specific examples of the modified antibodies and antigen-binding fragments thereof include nanobodies, AlbudAbs, dual affinity re-targeting (DARTs), bispecific T-cell engager (BiTEs), tandem diabodies (TandAbs), dual acting Fab (DAFs), two-in-one antibodies, small modular immunopharmaceuticals (SMIPs), fynomers fused to antibodies (FynomAbs), dual variable domain immunoglobulin (DVD-Igs), peptide modified antibodies (CovX-bodies), duobodies, and triomAbs, but is not limited thereto.

In a specific embodiment of the present disclosure, the antibody or antigen-binding fragment thereof according to the present disclosure comprises the aforementioned heavy chain variable region and light chain variable region and is selected from the group consisting of monoclonal antibodies, polyclonal antibodies, human antibodies, humanized antibodies, chimeric antibodies, scFv, Fab, F(ab), and F(ab)₂, but is not limited thereto.

Preferably, the antibody or antigen-binding fragment thereof may be a humanized antibody.

As used herein, the term "humanized antibody" refers to a chimeric immunoglobulin, an immunoglobulin chain or fragment thereof **(e.g.,** Fv, Fab, Fab', F(ab')₂ or other antigen-binding subsequences of the antibody) which comprises the minimal sequence derived from non-human immunoglobulin of non-human (e.g., mouse) antibodies. In most cases, humanized antibodies are human immunoglobulins (recipient antibodies) in which residues from a complementarity-determining region (CDR) of the recipient are replaced by residues from a CDR of a non-human species (donor antibody), such as mouse, rat or rabbit having desired specificity, affinity, and capacity. In some instances, Fv framework region (FR) residues of the human immunoglobulin are replaced by corresponding non-human residues. In addition, humanized antibodies may comprise residues which are found neither in the recipient antibody nor in the imported CDR or framework sequences. These modifications are made to further improve and optimize antibody performance. In general, the humanized antibody will comprise substantially all of at least one, and typically two, variable domains, in which all or substantially all of the CDR regions correspond to CDR regions of a non-human immunoglobulin and all or substantially all of the FR regions have sequences of FR regions of a human immunoglobulin sequence. The humanized antibody comprises at least a portion of an immunoglobulin constant region (Fc region), typically a constant region (Fc region) of a human immunoglobulin.

In another aspect thereof, the present disclosure provides a nucleic acid molecule comprising a nucleotide sequence encoding the antibody or antigen-binding fragment thereof that binds more specifically to the S-isomer of cotinine.

In an embodiment of the present disclosure, it is sufficient that the nucleic acid sequence encodes the amino acid sequence constituting the antibody or antigen-binding fragment thereof according to the present disclosure, and it is evident to those skilled in the art that it is not limited to any specific nucleotide sequence. The reason is that even if the nucleotide sequence undergoes mutation, the expression of the mutated nucleotide sequence into a protein may not cause a change in the protein sequence. This is called codon degeneracy. Therefore, the nucleotide sequence comprises nucleotide sequences comprising functionally equivalent codons, codons encoding the same amino acid (e.g., the number of codons for arginine or serine is six due to codon degeneracy), or codons encoding biologically equivalent amino acids.

In a specific embodiment of the present disclosure, the nucleic acid molecule of the present disclosure comprises (i) a nucleotide sequence of SEQ ID NO: 12 encoding a heavy chain variable region and a nucleotide sequence of SEQ ID NO: 13 encoding a light chain variable region; or (ii) a nucleotide sequence of SEQ ID NO: 14 encoding a heavy chain variable region and a nucleotide sequence of SEQ ID NO: 15 encoding a light chain variable region.

As used herein, the term "nucleic acid molecule" is intended to comprehensively encompass RNA molecules as well as DNA (gDNA and cDNA). Nucleotides, which account for a basic unit of nucleic acid molecules, include not only natural nucleotides but also analogues having modified sugar or base moieties (Scheit, Nucleotide Analogs, John Wiley, New York (1980); Uhlman and Peyman, Chemical Reviews, 90:543-584(1990)).

Considering biologically equivalent variations described in the foregoing, the nucleic acid molecule coding for the amino acid sequence accounting for the antibody or antigen-binding fragment of the present disclosure is construed to encompass sequences having substantial identity to them. Sequences having the substantial identity show at least 60%, particularly at least 70%, more particularly 80%, even more particularly at least 90%, most particularly at least 95% similarity to the nucleic acid molecule of this disclosure, as measured by using one of the sequence comparison algorithms for the sequences of the present disclosure aligned to any sequence, with maximum correspondence therebetween. Methods of alignment of sequences for comparison are well-known in the art. Various programs and alignment algorithms are disclosed in: Smith and Waterman, Adv. Appl. Math. 2:482(1981); Needleman and Wunsch, J. Mol. Bio. 48:443(1970); Pearson and Lipman, Methods in Mol. Biol. 24: 307-31(1988); Higgins and Sharp, Gene 73:237-44(1988); Higgins and Sharp, CABIOS 5:151-3(1989); Corpet et al., Nuc. Acids Res. 16:10881-90(1988); Huang et al., Comp. Appl. BioSci. 8:155-65(1992) and Pearson et al., Meth. Mol. Biol. 24:307-31(1994), but without limitations thereto.

Provided according to an aspect of the present disclosure is a recombinant vector comprising the nucleic acid molecule of the present disclosure described above.

As used herein, the term "vector" refers to a means for expressing a target gene in a host cell and is intended to encompass a variety of vectors comprising: plasmid vectors; cosmid vectors; and viral vectors such as bacteriophage vectors, adenovirus vectors, retrovirus vectors, and adeno-associated virus vectors.

According to a particular embodiment of the present disclosure, the nucleic acid molecule coding for an anti-CD19 antibody or an antigen-binding fragment thereof is operatively linked to a promoter in the vector of the present disclosure.

As used herein, "operatively linked" means that a nucleic acid expression control sequence (e.g., a promoter, a signal sequence, or an array of transcriptional regulatory factors) and a nucleic acid of interest are functionally linked so that the transcription and/or translation of the nucleic acid of interest can be governed by the control sequence.

The recombinant vector system of the present disclosure can be constructed by various methods known in the art. For concrete methods, reference may be made to Sambrook et al., Molecular Cloning, A Laboratory Manual, Cold Spring Harbor Laboratory Press (2001), which is incorporated in its entirety herein by reference.

The vector of the present disclosure may be typically constructed as a vector for cloning or expression. In addition, the vector of the present disclosure may be constructed with a prokaryotic cell or an eukaryotic cell serving as a host.

For example, when the vector of the present disclosure is an expression vector, with a eukaryotic cell serving as a host, advantage is taken of a promoter derived from the genome of a mammalian cell (e.g., metallothionein promoter, β-actin promoter, human hemoglobin promoter, and human muscle creatine promoter) or a promoter derived from mammalian viruses (e.g., adenovirus late promoter, vaccinia virus 7.5K promoter, SV40 promoter, cytomegalovirus promoter, tk promoter of HSV, mouse mammary tumor virus (MMTV) promoter, LTR promoter of HIV, promoter of Moloney virus, promoter of Epstein-Barr virus (EBV), and promoter of Rous sarcoma virus (RSV)), with a polyadenylated sequence commonly employed as a transcription termination sequence in the vector.

The vector of the present disclosure may be fused with the other sequences to facilitate the purification of the antibody expressed therefrom or antigen-binding fragment thereof. Examples of the fusion sequence include glutathione S-transferase (Pharmacia, USA), maltose binding protein (NEB, USA), FLAG (IBI, USA), and 6×His (hexahistidine; Quiagen, USA).

Meanwhile, the expression vector of the present disclosure comprises, as a selective marker, an antibiotic agent-resistant gene that is ordinarily used in the art, examples of which include resistant genes against ampicillin, gentamycin, carbenicillin, chloramphenicol, streptomycin, kanamycin, geneticin, neomycin, and tetracycline.

According to another aspect of thereof, the present disclosure provides a host cell transformed with the recombinant vector.

So long as it is capable of performing continuous cloning and expression while stabilizing the vector of the present disclosure, any host cell known in the art may be used, and for example, examples of eukaryotic host cells suitable for the vector include monkey kidney cells 7 (COS7), NSO cells, SP2/0, Chinese hamster ovary (CHO) cells, W138, baby hamster kidney (BHK) cells, MDCK, myeloma cell lines, HuT 78 cells, and HEK-293, but are not limited thereto.

As used herein, the term "transformed", "transduced", or "transfected" refers to pertaining to the delivery or introduction of a foreign nucleic acid into a host cell. The "transformed", "transduced", or "transfected" cells are cells into which a foreign nucleic acid is transformed, transduced, or transfected. Within the scope of the transformed, transduced, or transfected cells, the cells themselves and progeny cells thereof obtained through passages fall.

Provided according to another aspect of the present disclosure is a chimeric antigen receptor (CAR) comprising the antibody or antigen-binding fragment thereof that specifically binds to cotinine as described above.

The antibody of the chimeric antigen receptor or the antigen-binding fragment thereof is an anti-cotinine antibody or an antigen-binding fragment thereof.

In an embodiment of the present disclosure, the chimeric antigen receptor comprises an anti-cotinine antibody or antigen-binding fragment thereof, a hinge domain, a transmembrane domain, and a signaling domain, which are linked sequentially.

As used herein, the term "chimeric antigen receptor (CAR)" refers to an artificially constructed hybrid protein or polypeptide comprising a target binding domain linked to an effector cell-signaling or effector cell-activating domain (e.g., T-cell signaling or T-cell activating domain). In general, chimeric antigen receptors have the ability to redirect T-cell specificity and reactivity toward a selected target in a non-MHC-restricted manner, exploiting the antigen-binding properties of monoclonal antibodies. The non-MHC-restricted antigen recognition gives T-cells expressing CAR the ability to recognize an antigen independent of antigen processing, thus bypassing a major mechanism of tumor escape. Moreover, when expressed in T-cells, CAR advantageously does not dimerize with endogenous T-cell receptor (TCR) alpha and beta chains.

Specifically, the chimeric antigen receptor of the present disclosure refers to as a "switchable chimeric antigen receptor" (sCAR). The extracellular domain of a conventional classical chimeric antigen receptor (CAR) typically comprises an antibody or an antigen-binding fragment of an antibody that directly targets a specific antigen on the surface of a target cell. However, the extracellular domain of the chimeric antigen receptor of the present disclosure comprises an antibody or an antigen-binding fragment thereof that specifically binds to cotinine (specifically, cotinine conjugated to a switch molecule), targeting cotinine rather than directly targeting the surface antigen of the target cell.

The term "hinge domain" refers to a domain that connects the anti-cotinine antibody or fragment thereof to the transmembrane domain. It may comprise cysteine residues, and these cysteine residues may be involved in the binding of the hinge domain to the antibody. For example, the hinge domain may be derived from CD8 or CD28, or from IgG1, IgG2, IgG4, or IgGD, the extracellular domain of a killer immunoglobulin-like receptor (KIR), or combinations thereof, but is not limited thereto.

The term "transmembrane domain" refers to the part that connects the hinge domain and the signaling domain of the chimeric antigen receptor. The transmembrane domain can span the cell membrane so that the anti-cotinine antibody or antigen-binding fragment thereof in the chimeric antigen receptor is located on the cell surface, and the signaling domain is located inside the cell. The transmembrane domain may be selected from the group consisting of the alpha, beta, or zeta chains of T-cell receptor, CD27, CD28, CD3 epsilon (ε), CD45, CD4, CD5, CD8 (CD8α), CD9, CD16, CD22, CD33, CD37, CD64, CD80, CD86, CD134, CD137, and CD154, but with no limitations thereto.

The term "intracellular signaling domain" refers to the part located inside the cell membrane of an immune cell, specifically in the cytoplasm, which activates the immune response of the immune cell by transmitting a signal into the cell when the antibody or antigen-binding fragment thereof comprised in the extracellular domain binds to the target antigen.

The intracellular signaling domain of the present disclosure may be at least one selected from the group consisting of CD3 zeta (ζ), CD3 gamma (γ), CD3 delta (δ), CD3 epsilon (ε), FcR gamma, FcR beta, CD5, CD22, CD79a, CD79b, and CD66d, with preference for CD3 zeta (ζ), but is not limited thereto.

Additionally, the intracellular signaling domain of the present disclosure may further comprise a co-stimulatory domain, which may be at least one selected from the group consisting of ligands that specifically bind to CD2, CD7, CD27, CD28, CD30, CD40, 4-1BB (CD137), OX40 (CD134), ICOS, LFA-1, GITR, MyD88, DAP1, PD-1, LIGHT, NKG2C, B7-H3, and CD83, but with no limitations thereto.

Furthermore, the chimeric antigen receptor of the present disclosure may comprise modified forms of the antibodies and domains described above. These modifications can be made by substituting, deleting, or adding one or more amino acids to the wild-type amino acid sequences of the antibodies and domains, provided that the modifications do not alter their functions. Typically, the substitutions can be alanine or conservative amino acid substitutions that do not affect the overall charge, polarity, or hydrophobicity of the protein.

Another aspect of the present disclosure provides a chimeric antigen receptor effector cell expressing the chimeric antigen receptor comprising the antibody or antigen-binding fragment thereof that specifically binds to cotinine as described above.

The term "effector cell", as used herein, refers to a cell transformed with a vector comprising a gene coding for a chimeric antigen receptor polypeptide, meaning a cell that expresses the introduced chimeric antigen receptor on the cell membrane thereof.

In one embodiment of the present disclosure, an effector cell refers to an immune cell of hematopoietic origin responsible for the initiation and/or execution of innate and/or adaptive immune responses.

The immune cells according to the present disclosure may be dendritic cells, killer dendritic cells, mast cells, T cells, NKT cells, regulator T cells, NK cells, or B cells, and may be obtained from bone marrow, peripheral blood, peripheral blood mononuclear cells, or umbilical cord blood.

In an embodiment of the present disclosure, the cell may be an allogeneic or autologous cell with regard to the subject to be administered.

In a specific embodiment of the present disclosure, the effector cell is an immune cell expressing the chimeric antigen receptor on its surface, which may be a T cell.

In an embodiment of the present disclosure, the effector cell expresses the chimeric antigen receptor in the form where the antibody or antigen-binding fragment thereof according to the present disclosure is linked thereto, thereby targeting a cotinine-labeled conjugate. At this time, the binding of the effector cell and the cotinine-labeled conjugate may be an antigen-antibody binding between the anti-cotinine antibody or antigen-binding fragment thereof in the chimeric antigen receptor and cotinine.

Additionally, the effector cell may be activated by binding to the cotinine-labeled conjugate. Here, the cotinine-labeled conjugate serves as a switch molecule that regulates the activation of the effector cell. The activation of the effector cell includes exhibiting cytotoxic functions against target cells, cytokine secretion, or combinations thereof.

In a specific embodiment of the present disclosure, the chimeric antigen receptor effector cell is regulated in activation by a switch molecule comprising:
(a) the S-isomer of cotinine, and
(b) a targeting moiety that binds to a cell surface molecule on a target cell.

In a more specific embodiment of the present disclosure, the targeting moiety of the switch molecule is an antibody, an antigen-binding fragment of an antibody, or a target-binding polypeptide.

In an even more specific embodiment of the present disclosure, the switch molecule is a cotinine-conjugated affibody comprising the S-isomer of cotinine.

As used herein, the term "switch molecule" refers to an adaptor molecule in an effector cell using the chimeric antigen receptor, which separates the target recognition domain of the chimeric antigen receptor from the cell signaling domain of the chimeric antigen receptor and mediates this separation. The switch molecule allows for changing the target of the chimeric antigen receptor against heterogeneous targets or resistant tumors, or reducing the activation of chimeric antigen receptor effector cells by adjusting the administration of the switch molecule in case of excessive activation leading to side effects (Cao et al., Angew Chem Int Ed Engl. 2016 June 20; 55(26): 7520-7524).

As used herein, the term "target-binding polypeptide" refers to a non-immunoglobulin polypeptide molecule which exhibits binding affinity for a target antigen, like an antibody, but is not structurally relevant to an antibody. The target-binding polypeptides, also called antibody-like molecules or antibody mimetics, generally have a molecular weight of 3-20 kDa, unlike antibodies, which have a molecular weight of about 150 kDa. Examples of the target-binding polypeptide include, but are not limited to, an affibody derived from Z-domain of protein **A,** an affilin derived from gamma-B crystallin or ubiquitin, affimer derived from systatin, an alphabody, an anticalin derived from lipocalin, an avimer derived from a cell membrane receptor domain, DARPin derived from an ankyrin repeat motif, Fynomer derived from the SH3 domain of Fyn, a Kunits domain peptide derived from the Kunits domain of protease inhibitor, a monobody derived from the 10^{th} type III domain of fibronectin, and nanoCLAMP derived from carbohydrate binding module 32-2 of NagH in Clostridium perfringens.

Through various screening methods known in the art, such as phage display, ribosome display, etc., the target-binding polypeptide may be engineered to have binding affinity for any target antigen.

As used herein, the term "affibody" refers to the Z-domain of protein A from Staphylococcus aureus, which has affinity for IgG. An affibody is a small protein composed of 58 amino acid residues. In protein sequencing of the affibody molecule, 13 amino acids that form the binding surface with IgG can bind to various target antigens depending on the amino acid sequence thereof and can be randomly arranged to construct libraries. Similar to antibodies, affibody molecules capable of binding to various target antigens can be screened from libraries through screening methods, such as phage display and yeast two hybrid (Y2H). Affibody molecules specifically binding to HER2 and amyloid-β have been recently developed using characteristics of affibody molecules capable of binding to target antigens (Orlova et al. 2006, Cancer Res., Gronwall et al., 2007, J. Biotechnol.). When administered into the human body, the affibody molecules are systemically diffused and fast removed through renal clearance because they have a very small molecular weight of 6 kDa, compared to IgG, which generally has a molecular weight of 150 kDa. Therefore, affibody molecules are mainly applied to the research and development of diagnostic specimens (Goldstein R et al., 2013, Expert Rev Anticancer Ther.).

The affibody and cotinine, which are responsible for the constitution of the switch molecule in the present disclosure, are coupled to each other by chemical conjugation.

The chemical conjugation may be achieved by a chemical linker. Examples of the chemical linker include, but are not limited to, 1-ethyl-3-(3-dimethylaminopropyl)carbodiimide hydrochloride (EDC), dicyclohexyl carbodiimide (DCC), N-hydroxysuccinimide (NHS), N-hydroxysulfosuccinimide (Sulfo-NHS), imidoester-based linkers, and sulfosuccinimidyl 4-(N-maleimidomethyl)cyclohexane-1-carboxylate (Sulfo-SMCC). Any chemical linker that is typically used in the art may be employed without limitations.

In addition, the affibody and cotinine, which are responsible for the constitution of the switch molecule, may be connected indirectly via a linker.

A person skilled in the art could conceive that a linker may be used between functional moieties to be usually fused in the production of a fusion protein and would understand that there are different kinds of linkers having different characteristics, for example, a flexible amino acid linker, a non-flexible linker, and a cleavable amino acid linker. The linkers have been used for the purpose of increasing expression levels, improving biological activity, and enabling targeting, or modifying pharmacokinetics of the fusion protein, or in order to increase stability and improve folding property of the fusion protein.

According to another aspect thereof, the present disclosure provides an immunotherapeutic pharmaceutical composition comprising the chimeric antigen receptor effector cell as an active ingredient.

In one embodiment of the present disclosure, the immunotherapeutic pharmaceutical composition may further comprise the switch molecule described above to regulate the activity of the chimeric antigen receptor effector cell.

The term "immunotherapy", as used herein, refers to a method of treating cancer by helping the immune system to eliminate cancer. Immunotherapy can be either active or passive. Active immunotherapy includes i) cancer vaccine therapy in which cancer cells or a material produced from cancer cells are injected to the human body to boost the immune system, and ii) immune-modulating therapy in which immune-modulating agents such as cytokines (interferon, interleukin, etc.), growth factors, and so on are administered to activate specific leukocytes. Within passive immunotherapy are included the administration of therapeutic antibodies binding to specific cancer cells, and immune cell therapy. Examples of immune cell therapy include dendritic cell vaccine therapy, CAR-T (chimeric antigen receptor T cell) therapy, natural killer (NK) cell therapy, cytotoxic T lymphocyte (CTL) therapy, and adoptive cell transfer, but are not limited thereto. Herein, immune therapy refers mainly to the immune cell therapy.

The term "cancer", as used herein, is defined as disease characterized by the rapid and uncontrolled growth of aberrant cells. Cancer cells can spread locally or through the bloodstream and lymphatic system to other parts of the body. Examples of cancer include solid tumors and non-solid tumors **(e.g.,** hematological tumor such as leukemia and lymphoma).

The term "tumor", as used herein, refers to abnormal growth of tissue that may be benign, pre-cancerous, malignant, or metastatic. Specifically, solid tumors are typically abnormal tissue masses that do not contain cysts or liquid areas and can be benign or malignant. Different types of solid tumors are named according to the type of cells forming the tumor (e.g., sarcomas, carcinomas, and lymphomas).

Preferably, the diseases or conditions targeted by the immunotherapy of the present disclosure may be cancer, and the cancer may be solid cancer or hematological cancer. Here, the solid cancer may be lung cancer, colorectal cancer, prostate cancer, thyroid cancer, breast cancer, brain cancer, head and neck cancer, esophageal cancer, skin cancer, melanoma, retinoblastoma, thymic cancer, stomach cancer, colon cancer, liver cancer, ovarian cancer, uterine cancer, bladder cancer, rectal cancer, gallbladder cancer, biliary tract cancer, or pancreatic cancer. Additionally, the hematological cancer may be cancer of the blood or bone marrow, such as lymphoma, leukemia, or multiple myeloma.

Therefore, using the therapeutic system of the chimeric antigen receptor effector cells of the present disclosure, a pharmaceutical composition comprising effector cells expressing the chimeric antigen receptor can be administered to treat cancer, and efficient treatment can be achieved by additionally administering the switch molecule to regulate the activity of the effector cells.

Specifically, the switch molecule of the present disclosure includes an affibody that specifically binds to the HER2 antigen, and the diseases that can be treated using the aforementioned pharmaceutical composition are human and mammalian diseases associated with cells expressing HER2, as demonstrated in the Example section of the present disclosure.

Specifically, the switch molecule comprising a cotinine-conjugated anti-HER2 affibody and the effector cell expressing a chimeric antigen receptor targeting the cotinine of the switch molecule according to the present disclosure effectively kill the cancer cell lines SKOV3 and OVCAR3 that overexpresses HER2 on the surface thereof. Therefore, effector cells that express a chimeric antigen receptor comprising an antibody or antigen-binding fragment thereof more specifically binding to the S-isomer of cotinine can be advantageously used as active ingredients in a pharmaceutical composition for treatment of HER2-expressing carcinoma.

More specifically, the diseases targeted by the treatment may include breast cancer, stomach cancer, ovarian cancer, colorectal cancer, and endometrial cancer, but are not limited thereto.

Additionally, the pharmaceutical composition of the present disclosure may further comprise a switch molecule comprising a targeting moiety that binds to a cell surface molecule of the target cell, allowing for the manipulation of the target antigen of the targeting moiety to effectively prevent or treat a wider range of diseases.

A pharmaceutically acceptable carrier contained in the pharmaceutical composition of the present disclosure is one typically used in drug preparation and comprises, but not limited to, lactose, dextrose, sucrose, sorbitol, mannitol, starch, acacia rubber, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil.

The pharmaceutical composition of the present disclosure may further comprise a lubricant, a humectant, a sweetening agent, a favoring agent, an emulsifier, a suspending agent, and a preserving agent besides the components above. A suitable pharmaceutically acceptable carrier and a formulation are described in Remington's Pharmaceutical Sciences (19th ed., 1995).

The pharmaceutical composition of the present disclosure may be administered orally or parenterally, for example, intravenously, subcutaneously, intradermally, intramuscularly, intraperitoneally, intrasternally, intratumorally, topically, intranasally, interacerebrally, intracranially, intrapulmonarily, and rectally, but without limitations thereto.

Appropriate doses of the pharmaceutical composition of the present disclosure vary depending on various factors including the type of disease, the severity of the disease, the type and amount of the active ingredient and other components contained in the pharmaceutical composition, the type of formulation, the method of administration, the age, body weight, gender, and diet of the patient, the administration time, administration route, treatment duration, and concurrently used drugs. An ordinarily skilled practitioner can easily determine and prescribe an effective dose for desired treatment or prevention. According to a preferable embodiment of the present disclosure, the daily dose of the pharmaceutical composition of the present disclosure is 0.1-9 ×10⁵ to 0.1-9 ×10⁹ CAR-positive viable cells/kg. As used herein, the term "pharmaceutically effective amount" refers to an amount sufficient to prevent or treat the above-described diseases.

The pharmaceutical composition of the present invention may be formulated into a unit dosage form or may be prepared in a multi-dose container by using a pharmaceutically acceptable carrier and/or excipient according to a method that can be easily implemented by a person having an ordinary skill in the art to which the present invention belongs. Here, the formulation may be in the form of a solution in an oily or aqueous medium, a suspension, an emulsion, an extract, a pulvis, a suppository, a powder, granules, a tablet, or a capsule, and may further contain a dispersant or a stabilizer.

In addition to the aforementioned switch molecule, the pharmaceutical composition of the present disclosure may further comprise other pharmaceutically active agents or drugs, for example, chemotherapeutic agents such as asparaginase, busulfane, carboplatin, cisplatin, daunorubicin, doxorubicin, fluorouracil, gemcitabine, hydroxyurea, methotrexate, paclitaxel, rituximab, vinblastine, vincristine, and the like, but are not limited thereto.

Provided according to another aspect of the present disclosure is an immunotherapeutic pharmaceutical kit comprising the chimeric antigen receptor effector cells described above as an active ingredient.

In one embodiment of the present disclosure, the pharmaceutical kit may further comprise the switch molecule described above to regulate the activation of the chimeric antigen receptor effector cells.

Since the immunotherapeutic pharmaceutical kit of the present disclosure comprises the chimeric antigen receptor effector cells and switch molecule described in other aspects of the present disclosure, redundant details are incorporated by reference and common descriptions thereof are omitted in order to undue redundancy leading to the complexity of this specification.

### Advantageous Effects of Invention

The features and advantages of the present disclosure can be summarized as follows:
(a) The present disclosure provides an antibody or an antigen-binding fragment thereof that targets cotinine, with more specific binding affinity for the S-isomer of cotinine compared to the R-isomer;
(b) The present disclosure provides a chimeric antigen receptor (CAR) comprising the antibody or antigen-binding fragment thereof that targets cotinine, with more specific binding affinity for the S-isomer of cotinine, and effector cells expressing the chimeric antigen receptor;
(c) The present disclosure provides a switch molecule for activating the effector cells expressing the chimeric antigen receptor;
(d) The present disclosure provides an immunotherapeutic pharmaceutical composition and an immunotherapeutic pharmaceutical kit, each comprising the chimeric antigen receptor and the switch molecule; and
(e) The antibody or antigen-binding fragment thereof according to the present disclosure, which targets cotinine, with more binding affinity for the S-isomer of cotinine can induce superior cytotoxic activity to those induced by conventional antibodies. Furthermore, the improved specificity of the binding between the antibody and the switch molecule allows for more precise regulation of the action of chimeric antigen receptor effector cells, such as CAR-T cells, thereby effectively controlling side effects caused by the persistence of effector cells in the body.

### Brief Description of Drawings

FIG. 1 is a plot of quantitative specific binding of antibodies to cotinine as analyzed by ELISA.
FIG. 2 shows histograms of mean fluorescence intensity values of switche (cotinine-conjugated trastuzumab) binding to HER2 in HER2-expressing NCI-N87 cells, illustrating the cell binding ability of the switches.
FIG. 3 is a plot of quantitative binding affinity of hz33B2 as analyzed by ELISA.
FIG. 4 is a plot of the binding of the hz33B2 antibody as analyzed by flow cytometry.

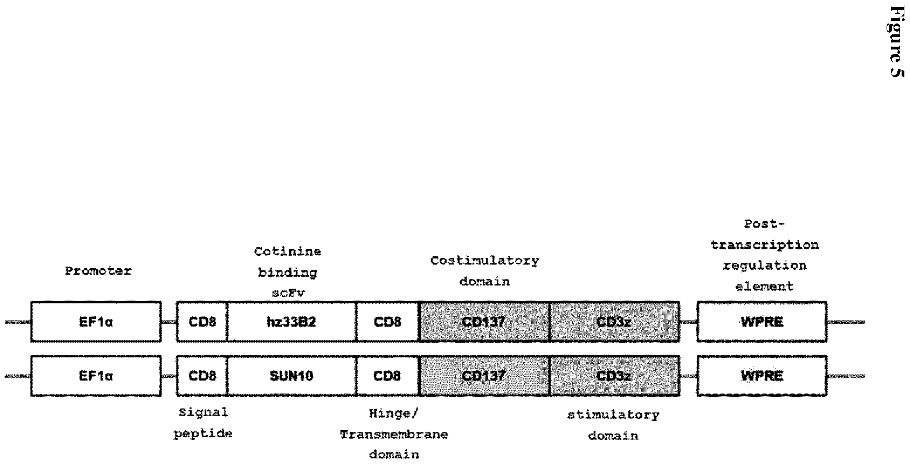

FIG. 5 shows schematic diagrams of CAR structures comprising the hz33B2 antibody and the SUN10 antibody used as a control, respectively.
FIG. 6 shows plots of cytotoxic effects of CAR-T comprising a cotinine-conjugated affibody and an anti-cotinine antibody on the HER2-overexpressing cell lines SKOV-3 and the HER2-expressing cell line OVCAR-3.
FIG. 7 is a plot of the binding of the hz33B2 antibody to switches conjugated with each cotinine isomer as analyzed by ELISA.
FIG. 8 shows flow cytometry charts illustrating the binding of switches conjugated with each cotinine isomer to CAR-T comprising the hz33B2 antibody.
FIG. 9 is a plot of cytotoxic effects of CAR-T comprising an anti-cotinine antibody and switches conjugated with each cotinine isomer on the HER2-overexpressing cell line SKOV-3.

### Best Mode for Carrying out the Invention

Hereinafter, embodiments of the disclosure will be described in detail with reference to the accompanying drawings. The following description of the embodiments is only to describe the disclosure in detail, and it will be apparent to one skilled in the art that the scope of the disclosure follows the gist of the disclosure and is not limited by the embodiments.

Throughout the description, the term "%" used to express the concentration of a specific material, unless otherwise particularly stated, refers to (wt/wt) % for solid/solid, (wt/vol) % for solid/liquid, and (vol/vol) % for liquid/liquid.

### EXAMPLES

### EXAMPLE 1: Development of Antibody Binding to Cotinine

### 1.1. Development of Antibody-Producing Cells Specifically Binding to Cotinine

### 1.1.1. Selection of antibody-producing cells specifically binding to cotinine

To develop an antibody specifically binding to cotinine, generation and selection were made of cells producing antibodies specifically binding to cotinine.

First, KLH (Thermo, 77600) was conjugated with cotinine using SMCC (Thermo, 22122) as a linker. KLH and SMCC were mixed at a weight ratio of 2:1 and reacted at room temperature for 1 hour to form a conjugate. The remaining SMCC was removed using an ultra-centrifugal filter. Cotinine was mixed at a ratio of 1:1 with the SMCC-conjugated KLH and allowed to conjugate overnight at 4°C. One hundred µg of KLH-cotinine was mixed with Freund's adjuvant (Sigma, F5506) and injected intraperitoneally into BALB/c mice (DBL. Co. Ltd). Two weeks later, an additional 100 µg of KLH-cotinine was diluted in PBS and injected. Three days later, the spleen of the mouse was excised, followed by isolating lymphocytes therefrom. The isolated lymphocytes were mixed with myeloma cells (SP2/0-Ag14, ATCC, CRL-1581) at a 5:1 ratio and fused using PEG-1500 (Roche, 10783641001). The fused cells were selectively cultured in a medium containing HAT supplement (Sigma, H0262) to selectively grow hybridoma cells.

### 1.1.2. Assay for antibody production ability of cells specifically binding to cotinine

ELISA assay was conducted to examine whether the selected hybridoma cells could produce antibodies binding to the antigen. First, BSA-cotinine with ability to detect antibodies specifically binding to cotinine was prepared using the same method as for the preparation of KLH-cotinine. BSA-cotinine was immobilized on a Costar 96-well plate (Corning, 3590) at a concentration of 1 µg/mL for 1 hour at room temperature. After washing three times with TBS-T (0.05% Triton X-100), the plate was blocked with 300 µL of TBS-T/SM (2% skim milk) for 30 minutes at room temperature. The blocked plate was washed thrice with TBS-T, added with the selected hybridoma culture supernatant, and incubated at 37°C for 1 hour to perform antigen-antibody reactions. In this regard, the SUN10 antibody (US 2008-0226650 A1) was used as a positive control. After three rounds of washing with TBS-T, a 5,000-fold dilution of anti-mIgG-HRP (Pierce, 31439) as a secondary antibody in TBS-T/SM was added to the plate and incubated at 37°C for 1 hour. Subsequently, the plate was again washed three times with TBS-T and then incubated with TMB (Surmodics, TMBC-1000-01) for 5 minutes at room temperature to develop color before stopping the color development by adding 1 N sulfuric acid (Duksan, 254). Absorbance was read at 450 nm using Victor X3 (PerkinElmer, 2030-0030). As a result, the selected hybridoma cells were observed to produce antibodies specifically binding to cotinine.

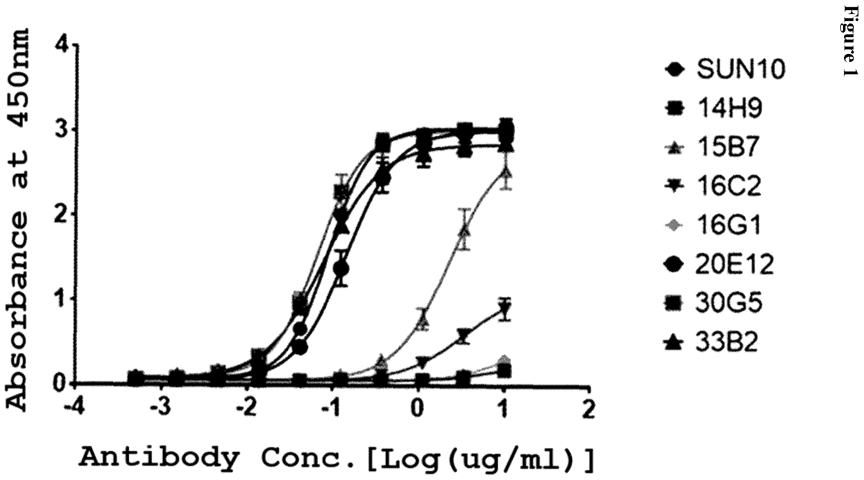

As shown in FIG. 1, 20E12, 30G5, and 33B2 were selected as antibodies specifically binding to BSA-cotinine.

### 1.2. Assay for Cell-Binding Ability of Antibodies Specifically Binding to Cotinine

To examine the cell-binding ability of the antibodies specifically binding to the selected BSA-cotinine, a cell-binding affinity assay was performed using NCI-N87 (ATCC, CRL5822) cancer cells expressing HER2 and an anti-HER2 switch (cotinine-conjugated trastuzumab).

### Production of Cotinine-Conjugated HER2-Targeting Switch Molecule

First, an anti-HER2 switch was produced by conjugating trans-4-cotininecarboxylic acid (Sigma, 33224-01-0) to trastuzumab in human IgG form through EDC reaction. The anti-HER2 switch was quantified using a protein assay dye (Bio-Rad, Cat. No. 500-0006), and the concentration and purity were measured by SDS-PAGE and Coomassie blue staining.

### Assay for Cell-Binding Ability Between Cotinine-Specific Antibodies and Targeting Switch

Then, NCI-N87 cells were prepared at a density of 5×10⁵ cells/tube, followed by centrifugation at 1,200 rpm for 3 minutes to collect the cells. The cells were washed with PBS containing 5% FBS and incubated with the anti-HER2 switch at 4°C for 1 hour. The cells were washed three times with 200 µl of PBS containing 5% FBS by centrifugation at 1,200 rpm for 3 minutes. Next, the cells were treated with the hybridoma culture supernatant at 4°C for 1 hour. The cells were again washed three times using the same method. Then, anti-mouse IgG-FITC (Jackson, 715-095-150) was added at 1 µg/mL to the cells before incubation at 4°C for 45 minutes in the dark. The cells were washed three times using the same method. The cell-binding ability of the selected antibodies was determined using a FACS device.

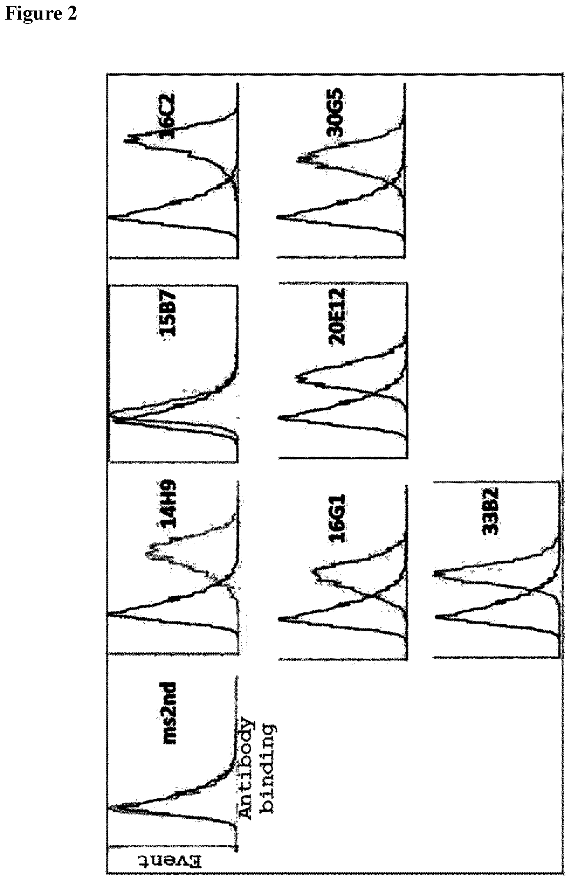

As shown in FIG. 2, the 33B2 antibody was observed to have the binding ability, along with the targeting switch. Table 1 below shows the sequence information for the selected 33B2 antibody.

**TABLE 1**

| Antibody Specifically Binding to Cotinine: 33B2 | | | |
|---|---|---|---|
| SEQ ID NO: | Name | Sequence | Type |
| 1 | 33B2_HCDR1 | SYWIQ | PRT |
| 2 | 33B2_HCDR2 | EIFPGTGTTYYNEKFKG | PRT |
| 3 | 33B2_HCDR3 | GGYYYDSRAWFAY | PRT |
| 4 | 33B2_LCDR1 | RSSTGTVTTSNYAN | PRT |
| 5 | 33B2_LCDR2 | GTNNRAP | PRT |
| 6 | 33B2_LCDR3 | ALWFSNHWV | PRT |
| 7 | 33B2_VH | EVQLQQSGAELVKPGASVKLSCKTSGYTFTSY WIQWIKQRPGQGLGWVGEIFPGTGTTYYNEKF KGKATLTIDTSSSTAYMQLSSLTSEDSAVYFC ARGGYYYDSRAWFAYWGQGTLVTVSA | PRT |
| 8 | 33B2_VL | DAVVTQESALTTSPGETVTLTCRSSTGTVTTS NYANWVQEKPDHLFTGLIGGTNNRAPGVPARF SGSLIGDEAALTITGAQTEDEAIYFCALWFSN HWVFGGGTKLTVLG | PRT |
| 12 | 33B2_VH | GAGGTGCAGCTTCAGCAGTCTGGAGCTGAGCT GGTGAAGCCTGGGGCTTCAGTGAAGCTGTCCT GCAAGACTTCTGGCTACACCTTCACCAGCTAC TGGATTCAGTGGATAAAACAGAGGCCTGGACA GGGCCTTGGGTGGGTTGGAGAGATATTTCCTG GAACTGGCACTACTTACTACAATGAGAAGTTC AAGGGCAAGGCCACACTGACTATAGACACATC CTCCAGCACAGCCTACATGCAGCTCAGCAGCC TGACCTCTGAGGACTCTGCTGTCTATTTCTGT GCAAGAGGGGGTTATTACTACGATAGTAGAGC CTGGTTTGCTTACTGGGGCCAAGGGACTCTGG TCACTGTCTCTGCAG | DNA |
| 13 | 33B2_VL | GATGCTGTTGTGACTCAGGAATCTGCACTCAC CACATCACCTGGTGAAACAGTCACACTCACTT GTCGCTCAAGTACTGGGACTGTTACAACTAGT AACTATGCCAACTGGGTCCAAGAAAAACCAGA TCATTTATTCACTGGTCTAATAGGTGGTACCA ACAACCGAGCTCCAGGTGTTCCTGCCAGATTC TCAGGCTCCCTGATTGGAGACGAGGCTGCCCT CACCATCACAGGGGCACAGACTGAAGATGAGG CAATATATTTCTGTGCTCTATGGTTCAGCAAC CATTGGGTGTTCGGTGGAGGAACCAAACTGAC TGTCCTAGGT | DNA |

### EXAMPLE 2: Construction and Production of Humanized Antibodies

To create a more druggable form of the selected antibody, the production of humanized antibodies was carried out.

### 2.1. Construction of Humanized Antibodies

Using the CDR grafting method, a humanized antibody of the mouse 33B2 antibody developed in Example 1 was created. The human antibody to receive the CDRs of the developed antibody was selected based on the nucleotide sequence similarity using IMGT/V-QUEST to identify the V and J genes of human germline antibody genes (Brochet, X. et al., Nucl Acids Res. 36:503-508 (2008)). The V and J genes for the heavy chain were selected as IGHV1-46*01 and IGHJ4*03, respectively, with similarities of 74.65% and 81.25%. The V and J genes for the light chain were selected as IGLV7-46*01 and IGLJ3*02, respectively, with similarities of 63.54% and 88.24%. For the synthesis, ClaI restriction enzyme sites were added in the forward direction while a NheI and a BsiWI restriction enzyme site were added in the reverse direction to the heavy and the light chain, respectively. Additionally, the signal sequence of Table 2 was added in the forward direction to the variable region to ensure that the humanized antibody could be secreted into the cell culture medium.

**TABLE 2**

| Signal peptide sequence of Mouse IgG kappa | | | |
|---|---|---|---|
| SEQ ID NO: | Name | Sequence | Type |
| 11 | Mouse IgG κ_Signal peptide | METDTLLLWVLLLWVPGSTG | PRT |

The synthesized variable regions were linked to the human constant regions Cκ and CH. The heavy chain region was TA cloned into the pcDNA3.3-TOPO (Invitrogen, K8300-01) vector, and the light chain region was TA cloned into the pOptiVEC-TOPO (Invitrogen, 12744-017) vector. Table 3 shows the sequence information of the humanized antibody (hz33B2) specifically binding to cotinine.

**TABLE 3**

| Humanized antibody specifically binding to cotinine: hz33B2 | | | |
|---|---|---|---|
| SEQ ID NO: | Name | Sequence | Type |
| 9 | hz33B2_VH | QVQLVQSGAEVKKPGASVKVSCKASGYTFTSY WIQWVRQAPGQGLEWMGEIFPGTGTTYYNEKF KGRVTMTIDTSTSTVYMELSSLRSEDTAVYYC ARGGYYYDSRAWFAYWGQGTLVTVSS | PRT |
| 10 | hz33B2_VL | QAVVTQEPSLTVSPGGTVTLTCRSSTGTVTTS NYANWVQQKPGQAPRGLIGGTNNRAPWTPARF SGSLLGDKAALTLSGAQPEDEAEYYCALWFSN HWVFGGGTKLTVLG | PRT |
| 14 | hz33B2_VH | CAAGTGCAGTTGGTCCAGTCAGGCGCAGAAGT AAAGAAACCAGGAGCGTCAGTTAAGGTCAGCT GCAAGGCGAGCGGCTATACGTTCACTTCATAT TGGATTCAGTGGGTACGGCAAGCGCCTGGTCA GGGCTTGGAATGGATGGGCGAAATTTTTCCTG GGACCGGGACGACATATTACAACGAAAAGTTC AAGGGAAGAGTTACCATGACGATCGATACATC TACCAGTACAGTCTACATGGAACTTAGCAGTC TTAGGAGCGAGGATACAGCAGTATATTATTGT GCGAGAGGTGGATACTACTATGATAGCCGGGC GTGGTTCGCGTATTGGGGCCAGGGGACACTTG TGACCGTCAGTAGT | DNA |
| 15 | hz33B2_VL | CAAGCGGTTGTGACACAAGAGCCCTCTTTGAC | DNA |
| | | TGTAAGCCCGGGTGGTACGGTCACACTTACAT GCCGCAGCTCTACCGGGACTGTAACAACCAGC AACTACGCGAACTGGGTACAACAGAAACCTGG ACAAGCGCCACGCGGTTTGATAGGAGGGACGA ATAATCGCGCCCCTTGGACCCCTGCGAGATTC TCTGGATCTTTGCTTGGGGACAAAGCAGCCCT TACCCTTAGCGGCGCACAGCCAGAGGATGAAG CTGAGTATTATTGCGCGCTGTGGTTTTCAAAC CACTGGGTGTTCGGCGGCGGCACGAAATTGAC CGTTCTTGGG | |

### 2.2. Production and Purification of Humanized Antibodies

The cloned vector was transiently transfected into FreeStyle^{™} 293F (Invitrogen, R790-07) animal cells using polyethyleneimine (Polysciences Inc., 23966), and the humanized antibodies were purified from the cell culture medium using Protein-A Ceramic HyperD F resin (PALL, 20078-028).

The purified humanized antibodies were quantified using a BCA assay (Pierce, 23227) and the concentration and purity were confirmed by SDS-PAGE followed by Coomassie blue staining.

### EXAMPLE 3: Assay for Binding Ability of Humanized Antibody (hz33B2)

### 3.1. Assay for Cotinine Binding Ability of Humanized Antibody (hz33B2)

To confirm the cotinine binding ability of the humanized antibody (hz33B2) developed in Example 2, ELISA and flow cytometry were performed using an anti-HER2 affibody conjugated with cotinine.

A plate coated with hHER2-ECD-His protein at a concentration of 2 µg/mL was washed three times with TBS-T (0.05% Triton X-100) and blocked with 200 µL of TBS-T/SM (3% skim milk) at room temperature for 120 minutes. After washing the blocked plate three times with TBS-T, the cotinine-conjugated anti-HER2 affibody (ZQAA1-Cot) was immobilized at a concentration of 2 µg/mL at room temperature for 1 hour. The plate was washed three times with TBS-T, and the humanized antibody was added at a 20 µg/mL starting concentration with 1/10 serial dilutions in 9 points, followed by incubation at room temperature for 1 hour. Here, the SUN10 antibody (US 2008-0226650 A1) was used as a positive control. Then, the secondary antibody, anti-hIgG-Fc-HRP (Invitrogen, H10007), was added and incubated at room temperature for 1 hour. Finally, the color development reaction was performed using TMB, and the OD450 value was measured using an ELISA reader.

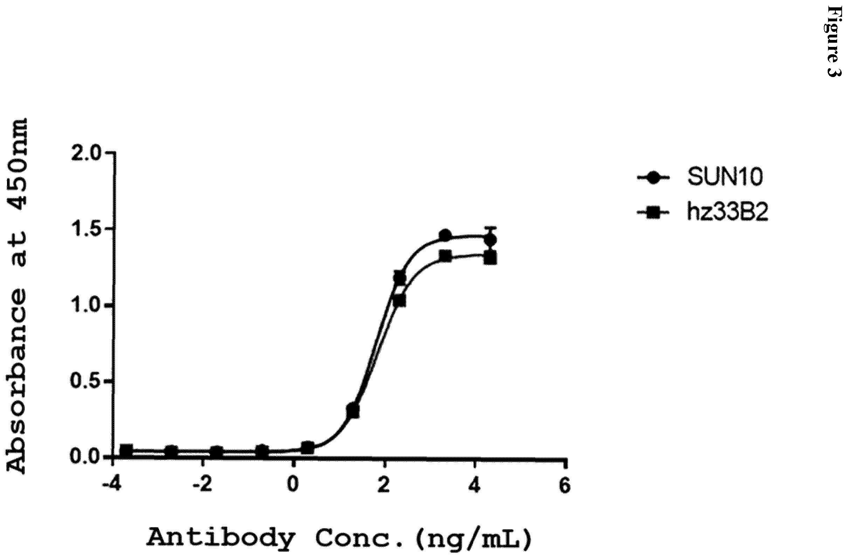

As shown in FIG. 3, it was observed that the humanized antibody (hz33B2) has high binding ability to cotinine.

### 3.2. Assay for Cell Binding Ability of Humanized Antibody (hz33B2)

Examination was made of the cell binding ability of the humanized antibody. In this regard, a cell binding affinity assay was performed using SKOV-3 cells expressing HER2 and a cotinine-conjugated anti-HER2 affibody to evaluate cell binding ability.

SKOV-3 cells (Korean Cell Line Bank, 30077) expressing HER2 were prepared at a density of 2×10⁵ cells/tube and centrifuged at 1,200 rpm for 3 minutes to collect the cells. Then, after washing three times with PBS containing 5% FBS, the cotinine-conjugated anti-HER2 affibody (ZQAA1-Cot) was incubated at a concentration of 2 µg/mL at 4°C for 1 hour. The cells were washed three times with PBS containing 5% FBS, and the humanized antibody was added at a 20 µg/mL starting concentration with 1/10 serial dilutions in 6 points, followed by incubation at 4°C for 1 hour. Here, the SUN10 antibody (US 2008-0226650 A1) was used as a positive control. Next, the cells were washed three times using centrifugation at 1,200 rpm for 3 minutes with 200 µL of PBS containing 5% FBS. Then, the secondary antibody, anti-human-IgG-BV421 (BD, 562581), was added at 1 µg/mL and incubated at 4°C for 30 minutes in the dark. After washing using the same method, the fluorescence intensity was measured using a FACS device.

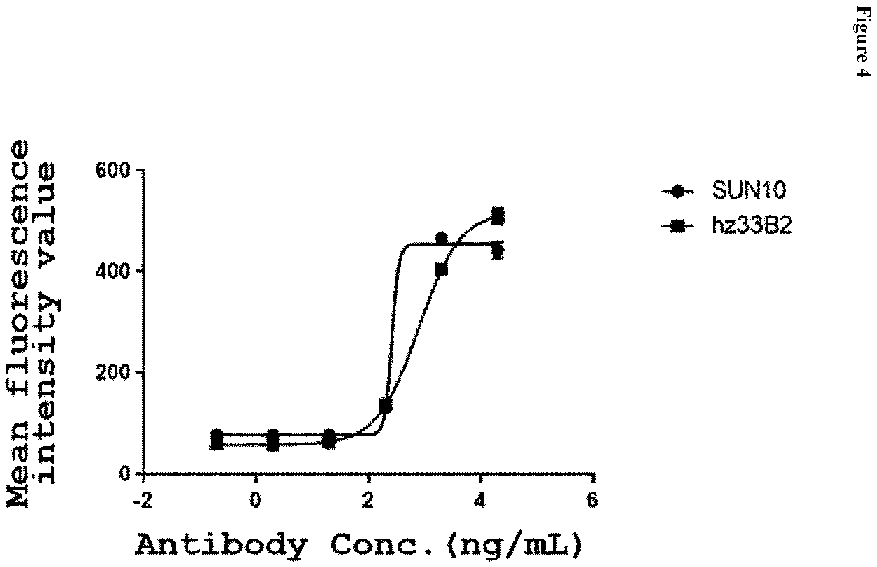

As shown in FIG. 4, it was observed that the humanized antibody (hz33B2) has high cell binding ability through binding with the cotinine-conjugated target switch.

### EXAMPLE 4: Development of CAR-T Comprising Humanized Anti-Cotinine Antibody and Assay for Activity thereof

### 4.1. Construction of Lentivirus Comprising Chimeric Antigen Receptor Linked to Anti-Cotinine Antibody Fragment

A chimeric antigen receptor (CAR) was developed using an anti-cotinine antibody fragment. The CAR consisted of a CD8 leader, an scFv form of anti-cotinine, a CD8 hinge and a transmembrane region, a CD137 cytoplasmic domain, and a CD3 zeta cytoplasmic domain. After codon optimization for the chimeric antigen receptor, it was inserted into the pLenti6-V5/DEST lentiviral vector (Invitrogen, V53306) modified with an EF-1 alpha promoter (see Table 4) using SpeI/XhoI digestion and ligation.

**TABLE 4**

| EF-1 alpha promoter sequence | | | |
|---|---|---|---|
| SEQ ID NO: | Name | Sequence | Type |
| 16 | EF-1 alpha promoter | GATGGAGTTTCCCCACACTGAGTGGGTGGAG ACTGAAGTTAGGCCAGCTTGGCACTTGATGT AATTCTCCTTGGAATTTGCCCTTTTTGAGTT TGGATCTTGGTTCATTCTCAAGCCTCAGACA GTGGTTCAAAGTTTTTTTCTTCCATTTCAGG TGTCGTGA | DNA |

The construct thus obtained was analyzed by nucleotide sequencing, and Table 5 provides the sequence information for the construct.

**TABLE 5**

| CAR construct sequence | | | |
|---|---|---|---|
| SEQ ID NO: | Name | Sequence | Type |
| 17 | CAR construct | ATCGAGGTGATGTACCCTCCTCCCTATCTCGA TAACGAGAAATCTAACGGCACCATCATCCATG TGAAAGGGAAACACCTCTGCCCTTCACCACTC TTCCCAGGTCCGAGCAAGCCAATTTATATCTG GGCACCGTTGGCGGGGACTTGCGGAGTGCTTT | DNA |
| | | TACTTTCACTGGTTATTACGCTGTACTGTAAA CGCGGTCGGAAGAAGCTCCTTTACATTTTCAA GCAGCCTTTTATGCGCCCAGTGCAGACCACAC AGGAGGAAGATGGCTGTAGTTGCAGATTTCCC GAGGAAGAAGAGGGAGGGTGTGAACTGAGAGT CAAATTCAGCCGTTCCGCTGATGCCCCAGCCT ATCAACAGGGGCAGAATCAACTGTATAATGAA TTGAATCTGGGCAGGAGAGAAGAATACGACGT CCTGGATAAGAGGCGAGGCAGAGACCCCGAGA TGGGCGGTAAACCCCGGCGGAAGAACCCCCAG GAAGGCCTGTACAACGAGCTGCAGAAGGACAA GATGGCTGAGGCCTACTCCGAAATAGGAATGA AGGGGGAGAGAAGGAGAGGCAAAGGACATGAC GGCCTGTACCAGGGACTGTCTACAGCTACTAA GGACACCTATGATGCATTGCACATGCAAGCCC TACCCCCTAGA | |

The prepared lentiviral construct was co-transfected, along with the plasmid pCMV-dR8.91 comprising gag, pol, and rev genes and comprising a nucleic acid coding for the virus envelope protein VSV-G (vesicular stomatitis Indiana virus G protein), into Lenti-X 293T cells (Takara Bio Inc., 632180). The transfection was performed using Lipofectamine 2000 (Invitrogen, 11668019) according to the manufacturer's protocol. After 72 hours, the culture medium containing the lentivirus was concentrated tenfold using a centrifugal filter device (Millipore, UFC910024) and stored at -80°C.

### 4.2. Production of Cytotoxic T Cell Presenting Anti-Cotinine Antibody Fragment-Comprising Chimeric Antigen Receptor on Surface

Using the lentivirus prepared in Example 4.1, cytotoxic T cells presenting the anti-cotinine antibody fragment-comprising chimeric antigen receptor on the surface thereof were produced.

First, human naive T cells were isolated and stimulated for 24 hours with Dynabeads^{™} Human T-Activator CD3/CD28 (Thermo Fisher Scientific, 11131D). The lentivirus was then transduced into the cells by incubation for 24 hours in the presence of polybrene (Sigma-Aldrich, H9268). Subsequently, the medium was replaced with medium containing IL-2 (Gibco, CTP0021), and the cells were cultured at 37°C in a 5% CO₂ atmosphere. The T cells presenting the anti-cotinine antibody fragment-comprising chimeric antigen receptor on the surface thereof were used in subsequent experiments within 24 hours of production.

### 4.3. Assay for Cytotoxic Effects Using CAR-T Comprising Anti-Cotinine Antibody

An experiment was conducted to examine whether the CAR-T cells produced in Example 4.2 and the cotinine-conjugated anti-HER2 affibody recognize HER2 on the cell surface and induce activation of the chimeric antigen receptor cells.

Specifically, lentivirus expressing GFP-Luciferase (Biosettia, GlowCell-16p-1) was introduced into HER2-overexpressing SKOV-3 cells and HER2-expressing OVCAR-3 cells to create the gene-introduced cell lines SKOV3-Luc and OVCAR3-Luc cells, respectively, and the cells were used in subsequent experiments. First, SKOV3-Luc and OVCAR3-Luc cell lines were dispensed at a density of 1×10⁴ cells per well in 96-well plates. Cytotoxic T cells were added to each well containing Luc cell lines at a ratio of 2:1 of SKOV3-Luc or OVCAR3-Luc : cytotoxic T cell (cell number ratio). The cotinine-conjugated anti-HER2 affibody was added at various concentrations (0.1, 1, 10, 100 nM) to the test groups treated with the Luc cells and cytotoxic T cells, and the plates were incubated under conditions of 5% CO₂ at 37°C for 24 hours. Here, SUN10-CAR-T cells (US 2008-0226650 A1) were used as a positive control. After incubation, the cytotoxic effect of the T cells was analyzed by measuring luciferase activity with the aid of the Bio-Glo Luciferase assay system (Promega, G7941). This was done by lysing the remaining SKOV3-Luc or OVCAR3-Luc cell lines with 3X Lysis buffer (75 mM Tris(pH 8.0), 30% glycerol, 3% Triton X-100) and reacting the eluted luciferase with the substrate. The lysis ratio was determined based on the signal from wells cultured with only the Luc cell lines as the reference (100%).

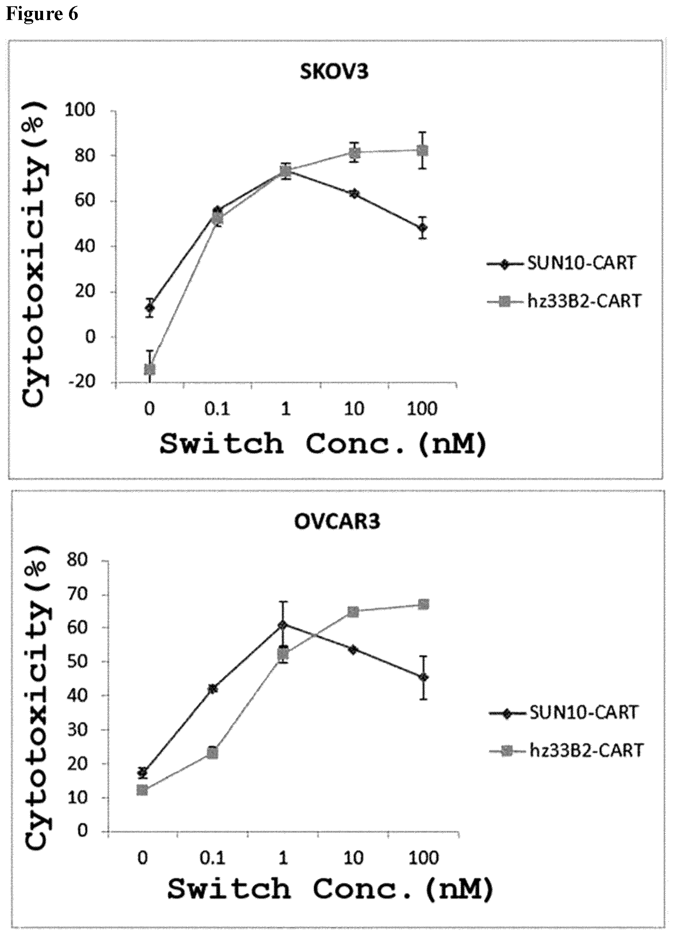

As shown in FIG. 6, it was confirmed that the cytotoxic effect increased with higher concentrations of the cotinine-conjugated affibody in all cell lines. Notably, it was observed that the cytotoxic effect was comparable to or superior to that of the previously reported SUN10-CAR-T (US 2008-0226650 A1) at higher concentrations.

### EXAMPLE 5. Assay for Binding Ability and Cytotoxic Activity of CAR-T Based on Cotinine Isomers

Since cotinine exists in a form mixed with two isomers, it was necessary to confirm whether the developed anti-cotinine binding antibody exhibits different binding characteristics and cytotoxic activity for each cotinine isomer.

### Preparation of Anti-HER2 Affibody Based on Cotinine Isomers

The cotinine racemate comprising two isomers was separated into two single isomers using a chiral column. The isolated isomers were determined to be the R-isomer and S-isomer through crystal structure analysis. The isolated isomers were conjugated to the anti-HER2 affibody ZQAA1 to synthesize two switches: ZQAA1-Cot(R) and ZQAA1-Cot(S).

### 5.1. Assay for CAR-T Binding Ability Based on Cotinine Isomers

CAR-T was analyzed for binding ability to each of the anti-HER2 switches comprising different cotinine isomers, using ELISA and flow cytometry.

### 5.1.1. ELISA for CAR-T Binding Ability

First, the binding ability of CAR-T to the two types of anti-HER2 switches was analyzed using ELISA.

A plate coated with hHER2-ECD-His protein at a concentration of 2 µg/mL was washed three times with TBS-T (0.05% Triton X-100) and blocked with 200 µL of TBS-T/SM (3% skim milk) at room temperature for 120 minutes. After washing the blocked plate three times with TBS-T, two anti-HER2 switches comprising respective different cotinine isomers were immobilized at a concentration of 2 µg/mL at room temperature for 1 hour. After washing the plate three times with TBS-T, the humanized antibody was added at a starting concentration of 20 µg/mL with 1/10 serial dilutions in 9 points and incubated at room temperature for 1 hour. Anti-hIgG-Fc-HRP (Invitrogen, H10007) was added as a secondary antibody and incubated at room temperature for 1 hour, followed by the color development reaction using TMB. The OD450 value was measured using an ELISA reader.

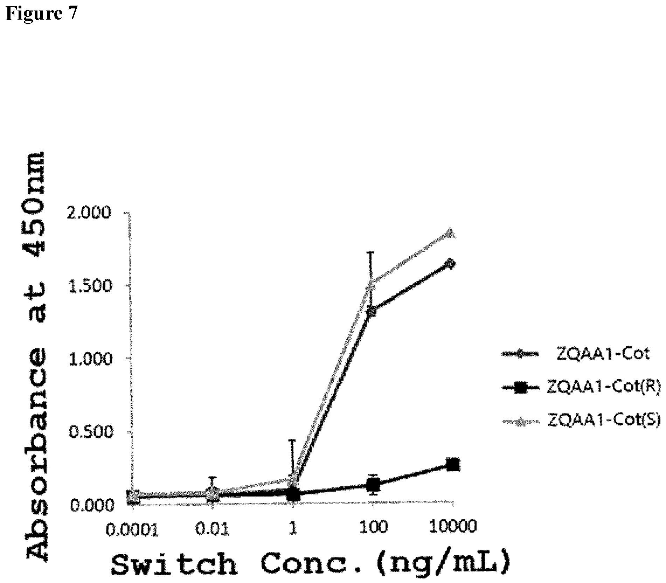

As shown in FIG. 7, it was observed that CAR-T comprising the anti-cotinine antibody of the present disclosure exhibited high binding ability to the anti-HER2 switch (ZQAA1-Cot(S)) conjugated with the S-isomer of cotinine. Compared to the results for the anti-HER2 switch (ZQAA1-Cot) conjugated with racemic cotinine, the binding ability was at the same or higher level, with slightly higher binding ability at high concentrations for the S-type cotinine isomer.

On the other hand, it was observed that the CAR-T comprising the anti-cotinine antibody hardly bound to the anti-HER2 switch (ZQAA1-Cot(R)) conjugated with the R-isomer of cotinine, demonstrating that the CAR-T of the present disclosure specifically binds to the S-isomer of cotinine.

### 5.1.2. Flow Cytometry Assay for CAR-T Binding Ability

Next, flow cytometry was performed to examine whether of the two types of anti-HER2 switches bind to the CAR-T comprising the anti-cotinine antibody.

The h33B2-CART produced in Example 4.2 was prepared at a density of 2×10⁵ cells/tube and centrifuged at 1,200 rpm for 3 minutes to collect the cells. After washing with PBS containing 5% FBS, the two types of anti-HER2 switches were each added at a concentration of 2 µg/mL and incubated at 4°C for 1 hour. After washing the cells in the same way, HER2-ECD-Fc was added at a concentration of 2 µg/mL and incubated at 4°C for 1 hour. The cells were washed three times with PBS containing 5% FBS by centrifugation at 1,200 rpm for 3 minutes. Then, 2 µL of anti-CD3-APC-H7 (BD, 560176), 2 µL of anti-human-Fc-PE (BioLegend, 410708), and 5 µL of 7-AAD (BD, 559925) were added to the cells and incubated at 4°C for 1 hour in the dark. The cells were washed three times in the same manner and the fluorescence intensity was measured using a FACS device.

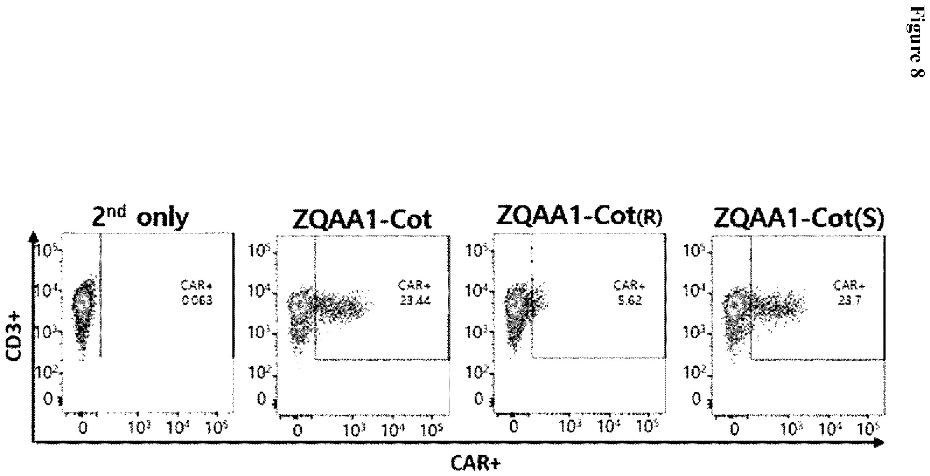

As shown in FIG. 8, it was found that the CAR-T comprising the anti-cotinine antibody of the present disclosure exhibited high binding ability to the anti-HER2 switch (ZQAA1-Cot(S)) conjugated with the S-isomer of cotinine. In particular, compared to the anti-HER2 switch (ZQAA1-Cot) conjugated with racemic cotinine, the binding ability was at the same or higher level, while almost no binding ability was observed for the R-isomer of cotinine. This was consistent with the results of the ELISA assay.

### 5.2. Assay for Cytotoxic Activity of CAR-T Based on Cotinine Isomers

The cytotoxic activity of CAR-T comprising the anti-cotinine antibody of the present disclosure was confirmed using the two types of anti-HER2 switches. The cytotoxic activity was evaluated using the same method as in Example 4.3 with the hz33B2-CART produced in Example 4.2.

Specifically, SKOV3-Luc cells were dispensed at a density of 1×10⁴ cells per well in 96-well plates. Cytotoxic T cells were added to each well containing the Luc cell lines at a ratio of 1:2 of SKOV3-Luc cells : cytotoxic T cells. The two types of anti-HER2 affibodies were added at various concentrations (0.0001, 0.001, 0.01, 0.1, 1, 10, and 100 nM) to the test groups treated with the Luc cell line and cytotoxic T cells and incubated under conditions of 5% CO₂ at 37°C for 24 hours. After incubation, the cytotoxic T cells was analyzed for cytotoxicity by measuring luciferase activity using the Bio-Glo Luciferase assay system (Promega, G7941). After incubation of SKOV3-Luc cells, cotinine-conjugated affibody, and Luc cells, the cytotoxic effect was measured by lysing the remaining SKOV3-Luc cell lines with 3X lysis buffer (75 mM Tris(pH 8.0), 30% glycerol, 3% Triton X-100) and reacting the eluted luciferase with the substrate. The lysis ratio was determined based on the signal from wells cultured with only the Luc cell lines as the reference (100%).

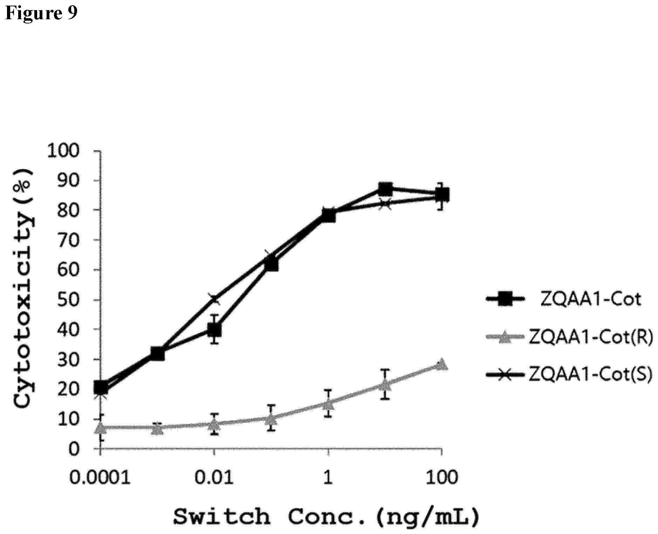

As shown in FIG. 9, it was observed that the anti-HER2 switch (ZQAA1-Cot(S)) conjugated with the S-isomer of cotinine exhibited excellent cytotoxic activity. In particular, the data showed high cytotoxic activity at the same level as the anti-HER2 switch (ZQAA1-Cot) conjugated with racemic cotinine. On the other hand, the cytotoxic activity for the R-isomer of cotinine showed a slightly increased activity only at high concentrations but remained significantly lower.

Therefore, it was found that the anti-cotinine antibody of the present disclosure exhibits differences in cotinine binding ability and cytotoxic activity of CAR-T based on the type of cotinine isomer.

## Claims

1. An antibody or an antigen-binding fragment thereof, binding more specifically to an S-isomer of cotinine than to an R-isomer of cotinine.

2. The antibody or antigen-binding fragment thereof according to claim 1, wherein the antibody of antigen-binding fragment thereof comprises:
a heavy chain variable region comprising HCDR1 with the amino acid sequence of SEQ ID NO: 1, HCDR2 with the amino acid sequence of SEQ ID NO: 2, and HCDR3 with the amino acid sequence of SEQ ID NO: 3; and
a light chain variable region comprising LCDR1 with the amino acid sequence of SEQ ID NO: 4, LCDR2 with the amino acid sequence of SEQ ID NO: 5, and LCDR3 with the amino acid sequence of SEQ ID NO: 6.

3. The antibody or antigen-binding fragment thereof according to claim 1, wherein the antibody or antigen-binding fragment thereof comprises:
(i) a heavy chain variable region comprising the amino acid sequence of SEQ ID NO: 7 and a light chain variable region comprising the amino acid sequence of SEQ ID NO: 8; or
(ii) a heavy chain variable region comprising the amino acid sequence of SEQ ID NO: 9 and a light chain variable region comprising the amino acid sequence of SEQ ID NO: 10.

4. The antibody or antigen-binding fragment thereof according to claim 1, wherein the antibody or antigen-binding fragment thereof is selected from the group consisting of a monoclonal antibody, a polyclonal antibody, a human antibody, a humanized antibody, a chimeric antibody, scFv, Fab, F(ab), and F(ab)₂, each comprising a heavy chain variable region and a light chain variable region.

5. A nucleic acid molecule, comprising a nucleotide sequence encoding the antibody or antigen-binding fragment thereof according to any one of claims 1 to 4.

6. The nucleic acid molecule according to claim 5, wherein the nucleic acid molecule comprises:
(i) a nucleotide sequence of SEQ ID NO: 12 encoding a heavy chain variable region and a nucleotide sequence of SEQ ID NO: 13 encoding a light chain variable region; or
(ii) a nucleotide sequence of SEQ ID NO: 14 encoding a heavy chain variable region and a nucleotide sequence of SEQ ID NO: 15 encoding a light chain variable region.

7. A recombinant vector comprising the nucleic acid molecule according to claim 5.

8. A host cell comprising the recombinant vector according to claim 7.

9. A chimeric antigen receptor, comprising the antibody or antigen-binding fragment thereof that binds more specifically to an S-isomer of cotinine according to any one of claims 1 to 4.

10. A chimeric antigen receptor effector cell expressing a chimeric antigen receptor comprising the antibody or antigen-binding fragment thereof that binds more specifically to an S-isomer of cotinine according to any one of claims 1 to 4.

11. The chimeric antigen receptor effector cell according to claim 10, wherein the activation of the chimeric antigen receptor effector cell is regulated by a switch molecule comprising:
(a) the S-isomer of cotinine, and
(b) a targeting moiety that binds to a cell surface molecule on a target cell.

12. The chimeric antigen receptor effector cell according to claim 11, wherein the targeting moiety as a constituent of the switch molecule for regulating activation is an antibody, an antigen-binding fragment of an antibody, or a target-binding polypeptide.

13. The chimeric antigen receptor effector cell according to claim 12, wherein the switch molecule for regulating activation is a cotinine-conjugated affibody comprising the S-isomer of cotinine.

14. An immunotherapeutic pharmaceutical composition comprising the chimeric antigen receptor effector cell according to claim 10 as an active ingredient.

15. An immunotherapeutic pharmaceutical kit comprising the chimeric antigen receptor effector cell according to claim 10 as an active ingredient.
